Europäisches Patentamt

European Patent Office

Office européen des brevets

(11) Publication number: **0 614 085 A1**

# EUROPEAN PATENT APPLICATION

(21) Application number: **94250049.7**

(51) Int. Cl.5: **G01N 33/18**, G01N 21/31

(22) Date of filing: **28.02.94**

(30) Priority: **03.03.93 US 25694**

(43) Date of publication of application:
**07.09.94 Bulletin 94/36**

(84) Designated Contracting States:
**AT BE CH DE DK ES FR GB GR IE IT LI LU MC NL PT SE**

(71) Applicant: **W.R. Grace & Co.-Conn.**
**Grace Plaza,**
**1114 Avenue of the Americas**
**New York, New York 10036-7794 (US)**

(72) Inventor: **Tippett, Roger John Arthur**
**1211 Prairie Brook Drive,**
**Apt. 3B**
**Palatine, Ill. 60067 (US)**
Inventor: **Richardson, John**
**329 East Everett Avenue**
**Crystal Lake, Ill. 60014 (US)**

(74) Representative: **UEXKÜLL & STOLBERG**
**Patentanwälte**
**Beselerstrasse 4**
**D-22607 Hamburg (DE)**

(54) **A method for directly monitoring the concentrations of water treatment compositions in steam generating systems.**

(57) A method for directly measuring the concentration of one or more water treatment compositions in a steam generating system which comprises directly determining an absorbance or emission spectrum of the system water in the wavelenth range of from 200 to 2500 nm, and applying chemometrics algorithms to the absorbance or emission spectrum to determine the concentration of the water treatment compositions.

FIG. I

Field of the Invention

The present invention is directed to a method for monitoring the concentration of one or more components of water treatment compositions in steam generating systems and more particularly to a direct spectrometric method for quantifying the concentrations of oxygen scavengers, corrosion inhibitors, chelants, nitrates, polymers, neutralizing amines, phosphonates, scale inhibitors, tracers and/or other boiler water treatment compositions which have absorbance or emission spectra in the wavelength range of from 200 to 2500 nm by determining the spectra of the compositions in the foregoing range and then applying chemometric algorithms to the spectrum.

Background of the Invention

The operation and control of steam generating systems requires careful monitoring of the water treatment composition levels to assure proper continuous system protection. As used herein, the terminology "steam generating system" refers to any aqueous system where water is heated and circulated as steam or hot water in a continuous or semi-continuous stream and generally includes, but is not limited to boiler systems, desalinization units, evaporators, turbines, cookers such as those used in food processing, and the like.

Traditional analyses of steam generating systems to determine the concentrations of the treatment compositions contained therein has generally involved taking grab samples and performing independent analytical procedures for each component of interest. These procedures are often time consuming and involve significant delay between the time the sample is taken, the time the results of the analysis are obtained and finally the time the subsequent product dosage is adjusted.

These analyses are further complicated by the need to sample the steam generating system water in at least three different locations within the system, depending, of course, on the particular type of treating agent. These locations include the feed water, the continuous surface boiler water blowdown and the steam condensate.

For example, the concentration of oxygen scavenger in a steam-generating system, is typically measured in the feedwater, the boiler water blowdown and the steam condensate. The concentrations of polymeric dispersants are often measured in the feed water and boiler water blowdown. Typically the product feed rate for these compositions is adjusted based upon the respective analyses of the residual oxygen scavenger and dispersant present in the boiler water blowdown.

While oxygen scavengers have been measured on-line in steam generating systems, these analyses have heretofore required the use of reagents to develop a color intensity which is proportional to the concentration of the oxygen scavenger. The deficiencies of this analytical technology are: slow response time, errors due to colorimetric interferences, and the necessity of periodic replacement of reagents.

Another method which has been used for measuring oxygen scavenger concentration is a platinum/silversilver oxide cell. However, this technique requires the use of a caustic soda electrolyte which needs to be replenished periodically.

Other on-line analyses have been used to measure inert (e.g. tracer) compounds. Tracers involve an indirect measurement and an assumption that the ratio of tracer compound to active treatment components will remain constant and thus indirectly gives an indication of active product performance. However, active treatment components are depleted in steam generating systems due to chemical reaction, adsorption, thermal instability or volatility. For example, the demand for oxygen scavengers and polymers will be dependent upon, inter alia, the concentration of dissolved oxygen and water hardness respectively. The dissolved oxygen content of the boiler feed water prior to the addition of the chemical oxygen scavenger may vary widely depending upon the efficiency of the deaerator system. The water hardness concentration in the boiler feedwater will vary dependent upon the efficiency of the softener system in the pretreatment process. Thus, the use of tracers is ineffective for accurately monitoring product dosage levels. There is, therefore, a need for a rapid, direct method of monitoring active water treatment compositions in steam generating systems.

Brief Description of the Drawings

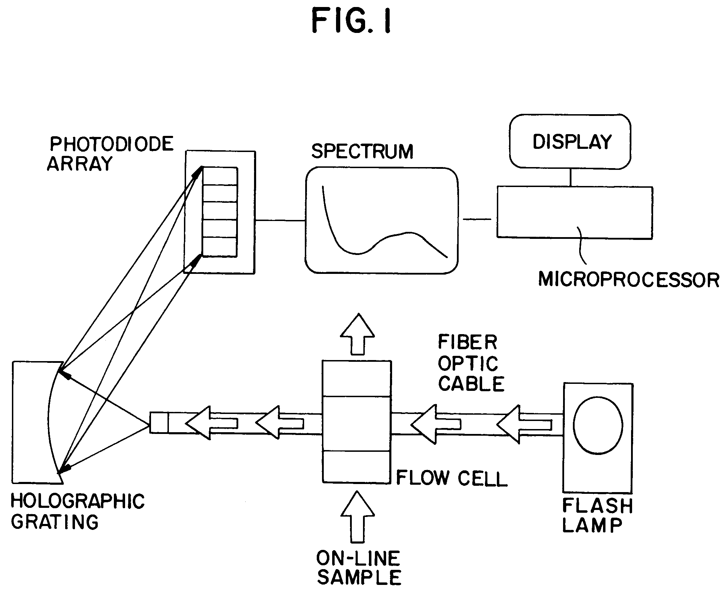

Figure 1 shows a schematic diagram of the array spectrometer and optrode arrangement.

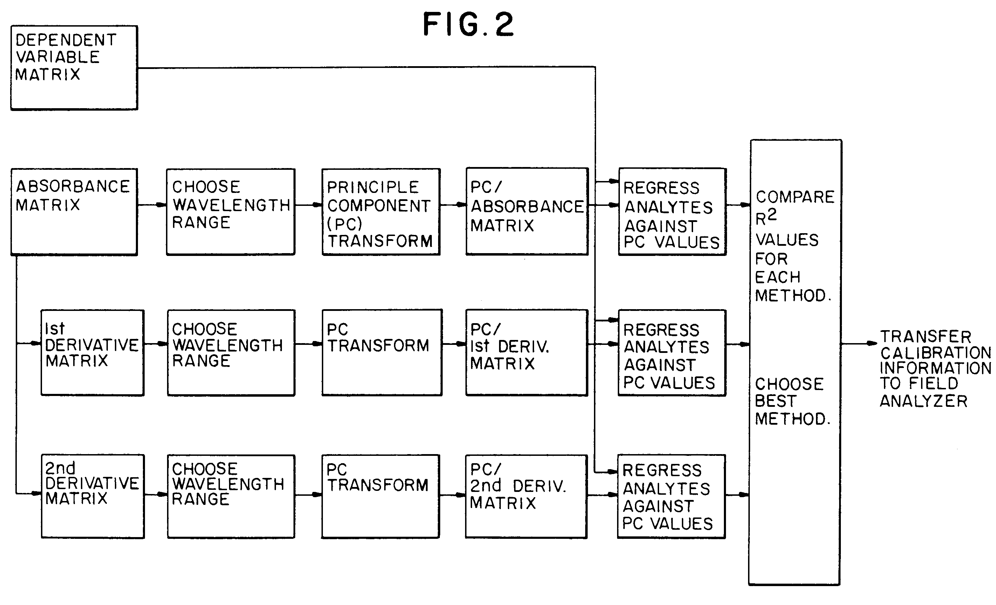

Figure 2 shows a block diagram of the calibration options available in this invention utilizing the principle of component regression analysis method.

EP 0 614 085 A1

## Summary of the Invention

It is an object of the present invention to provide a method for directly measuring the concentration of water treatment compositions in a steam generating system without the need for additional reagents, titrations or separation techniques and which is not generally affected by background interferences such as e.g. pH changes.

It is an object of this invention to provide a method for directly measuring levels of oxygen scavengers, polymeric dispersants and/or nitrates either individually or simultaneously in steam generating systems thus providing immediate feed back to adjust and control product feed rate.

It is another object of this invention to provide a method for identifying and/or quantifying low levels of a water treatment component having a weak ultraviolet, visible and/or near infrared response (absorbance or emission) in the presence of one or more water treatment components having strong ultraviolet, visible and/or near infrared response through the application of rotated principle component analysis to the spectrum of the system water containing these components.

In accordance with the present invention, there has now been provided a method for directly measuring the concentration of one or more water treatment compositions in a steam generating system which comprises directly determining an absorbance or emission spectrum of the system water in the wavelenth range of from 200 to 2500, and applying chemometrics algorithms to the absorbance or emission spectrum to determine the concentration of the water treatment compositions.

Also provided in accordance with the present invention is a method of determining cycles of concentration in a steam generating system which contains a monitorable component in the systems which is not actively consumed in the system water comprising monitoring the concentration of the component in a feed water stream and in a blowdown stream of the system and determining a ratio of the concentration of the component in the blowdown to the concentration of the component in the feedwater wherein said ratio equals the cycles of concentration.

Also provided in accordance with the present invention is a method for determining the effective dosage amount of a polymer to treat an aqueous system. It has now been found that by directly measuring the polymer concentration in the system and also determining the level of hardness in the feedwater, it is possible to more effectively control polymer dosage amounts. Accordingly, this embodiment of the invention is directed to a method for determining polymer dosage requirements by measuring the concentration of hardness in the feedwater with a hardness analyzer, generating a voltage analog which is proportional to the degree of hardness, measuring the polymer treatment concentration with a spectometer having a wavelength range of from 200 to 2500 nm, applying chemometrics algorithms to the spectrum to determine the concentration and generating a voltage analog which is proportional to the polymer treatment concentration, outputting the respective voltage analogs to a logic controller which is capable of calculating a ratio of these voltage analogs, and then comparing the calculated ratio to a predetermined standard operating range, and if the ratio is outside the standard operating range, increasing or decreasing the rate of polymer treatment feed until an acceptable ratio is obtained.

Also provided in accordance with the present invention is a method for determining loss of polymer treatment in steam generating systems which comprises measuring the concentration of polymer in the blowdown and comparing this to a theoretical polymer level wherein polymer loss can be determined by the following formula:

Polymer loss = Theoretical polymer level in blowdown - measured polymer level in blowdown

## Detailed Description

The present invention is directed to a novel method for directly monitoring the concentrations of one or more components of water treatment compositions in steam generating systems. The method, in general, involves directly determining an absorbance or emission spectrum of the system water containing the water treatment compositions in the ultraviolet, visible and/or near infrared wavelength range, and then applying chemometrics algorithms to the spectrum to determine the concentrations of the water treatment compositions. The method of the present invention is generally unaffected by background matrix interferences and thus does not require time consuming, off-line separation or derivitization techniques. In addition, the method of the present invention does not require or involve the use of additional colorizing agents, dyes, titrations or other typical indirect monitoring techniques.

The present monitoring method is of general applicability with respect to water treatment compositions used to treat steam generating systems provided, of course, that the particular water treatment composition

3

has a detectable absorbance or emission spectrum in the visible, ultraviolet and/or near infrared range (i.e., in a wavelength range of 200 to 2500 nm). For purposes of this invention, water treatment compositions are considered to be "detectable" if they have a chromophore with at least about 0.01 absorbance units (or a corresponding measureable response with an emissivity spectrometer) in the wavelength range of from 200 to 2500 nm under normal treatment dosages and preferably have between 0.1 to 1.5 absorbance units. Suitable monitorable water treatment compositions may easily be determined by preparing an aqueous solution of the composition in its normal dosage concentration, and then scanning the solution in a suitable spectometer in the range 200 to 2500 nm. If the spectrum exhibits an absorbance or emission maxima of 0.01 absorbance or emissivity units from a background spectrum (i.e., without the compound of interest), then this water treatment component is considered to be monitorable under this invention.

Particular water treatment components of interest include oxygen scavengers, oxygen scavenger catalysts, corrosion inhibitors, chelants, nitrates, tracers, scale inhibitors, neutralizing amines, polymers, and the like and mixtures thereof. Typical oxygen scavengers which have been found to be monitorable in accordance with the present invention, include sulfite, hydrazine, erythorbic acid, alkylhydroxyl amines such as e.g., diethylhydroxylamine, n-isopropylhydroxyl amine, and the like, hydroxyalkyl substituted hydroxyl amines including bis(2-hydroxyethyl)-hydroxylamine, bis(2-hydroxypropyl)-hydroxylamine, bis(2-hydroxybutyl)hydroxylamine and the like, precursors to hydroxylamines such as methylethylketoxime and the like, and mixtures thereof. Typical catalysts for oxygen scavengers include copper, hydroquinone, benzoquinone, 1,2-naphthoquinone-4-sulfonic acid, pyrogallol, t-butyl-catechol, and the like and mixtures thereof. Typical corrosion and/or scale inhibitors include nitrate, molybdate, neutralizing amines, phosphates, phosphonates, and the like and mixtures thereof. Typical chelants include nitrilotriacetic acid, ethylene diamine tetracetic acid and the like. Typical tracers include inert dyes having absorbance or emission spectra in the wavelength range of 200 to 2500 nm such as, 2-naphthalene sulfonic acid, lissamine FF, bromophenol, rhodamine, stilbene derivatives, sulfostyrene derivatives, propene, xanthene, fluoroscein, and the like and mixtures thereof. Typical polymers include, but are not limited to homo-polymers and copolymers containing one or more of the following recurring moieties, acrylates, methacrylates, acrylamides, methacrylamides, sulfonated styrenes, lignosulfonates, tannins, alkyl naphthalene sulfonates, and the like.

In accordance with the present invention, a steam generating system may be monitored at any convenient location in the system and is generally monitored in the feed water stream, the continuous blowdown stream and/or the steam condensate stream, and is preferably monitored in the continuous blowdown stream. It is also possible to monitor the system in the bottom blowdown stream. However, since this aqueous stream often contains large amounts of particulate matter, it is advisable to filter the bottom blowdown water stream prior to obtaining its spectrum.

Generally, any commercial grade UV, visible and/or near infrared spectrometer may be used in accordance with this invention. For example, it is possible to use fixed wavelength detectors where discrete elements are placed at specific wavelengths which generally correspond to the absorbance or emission maxima for the particular water treatment composition. Charged coupled device (CCD) analyzers may also be used. It is preferred that the spectrometer have a resolution of at least 10 nm, preferably 2 nm and most preferably 1 nm.

An array spectrometer having wavelengths within the range of from 200 to 2500 nm is preferred for use in this invention and most preferably has a wavelength range of from 200 to 800 nm. Instrument stability is an important consideration when operating in areas with a high potential for electrical and mechanical noise. The spectrometer is preferably designed to operate at 40°C to eliminate any varying temperature effects.

The spectrometer may be used to monitor off-line samples, or in a preferred embodiment, is equipped with an on-line fiber optic probe.

For on-line measurements a flow through optical chamber (optrode) is preferred. In these systems, light from a xenon flash lamp (or other suitable source) is transmitted to the optrode via a quartz fiber optic cable. The light is transmitted through the aqueous solution and collected in a second fiber optic cable which transmits the light to the diode array spectrometer. In the spectrometer the light is converted into an analog voltage for each pixel of the array. The array is then read by a computer and by subtracting a previously stored deionized water scan from the sample scan a true absorption spectrum is generated. The resultant spectrum is then processed by a chemometrics calibration algorithm to generate a quantitative multicomponent analysis for any and all of the water treatment compositions of interest.

Chemometrics is the application of statistical and pattern recognition techniques to chemical analysis. Quantitative estimates of chemical concentration in reagentless UV-vis-NIR spectroscopy are based on algorithms, the parameters of which are determined in calibration sequences called learning sets. Learning sets consist of a large number of known samples that are used to determine the parameters of the algorithms. The number of samples required depends on the complexity of the matrix, the number of spectroscopic interferences that are present, and the number of variables used in the algorithm. In general, the number of samples should be at least 10 times the number of independent variables employed. In the presence of known and unknown interferences, a multiple sample calibration averages out the effects of these interferences. The learning set solutions are prepared in a manner to typify the interferences and their variability that will be experienced in the steam generating system.

The invention uses a multi-sample calibration based on either principle component regression analysis or rotated principle component analysis of absorbance, and subsequent derivative data. The rotated principle component analysis method is preferred and involves a rotation of the principle components which allows the concentration of all the relevant information for a particular analyte into a single rotated principle component. The use of rotated principle components enables one to detect weak UV-vis-NIR species that would not normally be quantifiable using more conventional chemometric techniques.

The most accurate calibration method for each analyte may be determined by selecting the particular method having the highest coefficient of determination ($r^2$) value.

Another embodiment of this invention is directed to a method for determining the cycles of concentration in a steam generating system. Current methods of calculating cycles of concentration involve monitoring the chloride ion level in the system. This method has a relatively high degree of experimental error, and involves off-line wet chemical techniques which are time consuming and do not provide continuous results. It has now been found that the method of the present invention when used to monitor the level of a component having a low system demand such as e.g. nitrate levels or inert tracer levels in a steam generating system provides a continuous, accurate method for calculating the cycles of concentration.

Nitrate is usually present as a natural contaminant in boiler feed water and boiler water blowdown except in those steam generating systems where demineralization is used as the method of water softening. Since nitrate has a low system demand the concentration of nitrate can be monitored in the boiler feed water and boiler blowdown and thus provides an accurate method of calculating cycles of concentration wherein;

$$\text{Cycles of Concentration} = \frac{\text{Concentration } NO_3^- \text{ in blowdown}}{\text{Concentration } NO_3^- \text{ in feedwater}}$$

This method is suitable for calculating cycles of concentration in those steam generating systems that have a loss of nitrate which is generally less than 10% as measured against chloride ion or other stable component in the system. In those systems where nitrate loss exceeds 10%, or where it may be advantageous for other reasons, an inert tracer may be monitored in addition to or instead of the nitrate. Accordingly, cycles of concentrations can be calculated by monitoring the concentrations of tracer in the blowdown and in the feedwater, and substituting these concentrations into the above calculation, respectively.

Yet another embodiment of this invention is directed to a method of accurately determining polymer dosage requirements in a steam generating system. This method involves 1) measuring the degree of hardness of the feedwater with a hardness analyzer which generates a voltage analog which is proportional to the degree of hardness, 2) measuring the polymer treatment concentration in accordance with the present invention and generating a voltage analog which is proportional to the concentration, 3) outputting the respective voltage analogs to a logic controller which is capable of calculating a ratio of these voltage analogs, and then comparing the ratio to a predetermined standard operating range, and if the ratio is outside the standard operating range, increasing or decreasing the rate of polymer treatment feed until an acceptable ratio is obtained.

Another embodiment of this invention is directed to a method for determining loss of polymer in steam generating systems which comprises measuring the concentration of polymer in the blowdown and comparing this to a theoretical polymer level wherein polymer loss can be calculated by the following formula:

Polymer loss = Theoretical polymer level in blowdown - measured polymer level in blowdown

The determination of theoretical polymer level in the blowdown can be made by either 1) measuring the level of an inert tracer in the blowdown and which was dosed in a known proportion with the polymer and then multiplying this proportion by the concentration of tracer in the blowdown or 2) by directly measuring the level of polymer in the feedwater and then multiplying this value by the cycles of concentration.

Without further elaboration, it is believed that one of ordinary skill in the art using the foregoing detailed description can use the present invention to its fullest extent. The following examples are provided to illustrate the present invention in accordance with the principles of this invention, but are not to be construed as limiting the invention in any way except as indicated in the appended claims. All parts and percentages are by weight unless otherwise indicated.

Examples

Unless otherwise indicated, the following Operating Parameters were used in each of the following examples:

On-line analyzer

Resolution 2 nm Operating temperature 40°C

Solution path length 1.3 cm Static solution measurements

Chemometric Techniques

Learning set size (10-70) samples

Wavelength range - 26-30 wavelengths in the range 230-327 nm

Calibration based upon rotated principle component on absorbance, first derivative or second derivative spectrum.

Example 1

A calibration of a sodium styrene sulfonate methacrylic acid copolymer was prepared in demineralized water at pH 10 containing 10 ppm disodium phosphate (as $PO_4^{-3}$).

Concentration range of learning set - polymer - 0-5.0 ppm active.

Number of samples in learning set - 14

Wavelength range - 230 nm - 284 nm

This test set of samples was passed through the analyzer, the concentrations of the polymer were determined and a comparison made between the theoretical polymer value and the predicted value based upon the calibration.

Table 1

| Sample # | Theoretical Concentration ppm | Predicted Concentration ppm | Error % |
|---|---|---|---|
| 1 | 0.35 | 0.34 | 2.86 |
| 2 | 0.45 | 0.46 | 2.22 |
| 3 | 0.70 | 0.71 | 1.43 |
| 4 | 1.10 | 1.12 | 1.82 |
| 5 | 3.30 | 3.24 | 1.82 |

The foregoing results demonstrate that when only 1 analyte is present, there is an extremely good correlation between the predicted and the theoretical concentrations of the polymer.

Example 2

A calibration of a sodium styrene sulfonate methacrylic acid copolymer and sodium sulfite was prepared in demineralized water at pH 10 containing 10 ppm disodium phosphate.

Concentration - polymer 0-25 ppm (active)

Ranges in the learning set - sulfite 0-50 ppm (as $SO_3^{2-}$)

Number of samples in learning set - 26

The test set contained 4 samples of mixtures of the sodium styrene sulfonate/methacrylic acid copolymer and sodium sulfite.

Table 2

| Sample # | Polymer | | | Sulfite | | |
|---|---|---|---|---|---|---|
| | Theoretical Conc. ppm | Predicted Conc. ppm | % Error | Actual Conc. ppm | Predicted Conc. ppm | % Error |
| 1 | 2.40 | 2.38 | 0.94 | 30.20 | 29.80 | 1.28 |
| 2 | 8.00 | 7.75 | 2.74 | 20.20 | 20.84 | 3.18 |
| 3 | 12.00 | 11.89 | 0.84 | 12.00 | 12.28 | 2.32 |
| 4 | 16.00 | 15.89 | 0.67 | 34.00 | 34.17 | 0.51 |

\* The actual concentrations of sulfite in the learning set and test set samples was determined by iodometric titration. The reaction of oxygen with the sodium sulfite caused the actual concentration of sulfite to be lower than the theoretical value.

This example demonstrates that polymers and oxygen scavengers can be accurately measured simultaneously using the on-line analyzer.

Example 3

A calibration of a sodium styrene sulfonate/maleic acid copolymer and sodium sulfite was prepared in demineralized water at pH 10 containing 10 ppm disodium phosphate (as $PO_4^{3-}$).

7

Concentration ranges in the learning set:

Polymer 0-25 ppm (active)

Sulfite 0-50 ppm (as $SO_3{}^{2-}$)

Number of samples in the learning set - 31

The test set contained 6 samples of mixtures of the sodium styrene sulfonate/methacrylic acid copolymer and sodium sulfite.

Table 3

| Sample # | Polymer | | | | Sulfite | | |
|---|---|---|---|---|---|---|---|
| | Theoretical Conc. ppm | Predicted Conc. ppm | % Error | | Actual Conc. ppm | Predicted Conc. ppm | % Error |
| 1 | 2.00 | 1.91 | 4.50 | | 7.60 | 8.24 | 8.42 |
| 2 | 4.00 | 3.61 | 9.75 | | 39.10 | 37.92 | 3.02 |
| 3 | 8.00 | 7.66 | 4.25 | | 25.80 | 25.63 | 0.66 |
| 4 | 12.00 | 11.57 | 3.58 | | 16.40 | 17.12 | 4.39 |
| 5 | 16.00 | 15.42 | 3.63 | | 30.20 | 29.86 | 1.13 |
| 6 | 20.00 | 19.23 | 3.85 | | 9.50 | 10.17 | 7.05 |

*      The actual concentration of sulfite determined by iodometric titration.

This example demonstrates that mixtures of the sodium styrene sulfonate/maleic acid and sodium sulfite can be measured simultaneously with good accuracy.

Example 4

A calibration of a sodium styrene sulfonate methacrylic acid copolymer with sodium nitrate was prepared in demineralized water at pH 10 containing 10 ppm disodium phosphate (as $PO_4^{3-}$),

9

Concentration ranges in the learning set:
polymer 0-25 ppm (active)
nitrate 0-25 ppm (as $NO_3^-$)
Number of samples in the learning set - 30

The test set contained 2 samples of mixtures of the sodium styrene sulfonate/methacrylic acid copolymer and sodium nitrate.

Table 4

| Sample # | Theoretical Conc. ppm | Predicted Conc. ppm | % Error | Actual Conc. ppm | Predicted Conc. ppm | % Error |
|---|---|---|---|---|---|---|
| 1 | 3.00 | 2.61 | 13.10 | 21.00 | 22.60 | 7.60 |
| 2 | 22.00 | 21.40 | 2.70 | 15.00 | 16.23 | 8.15 |

This example demonstrates that mixtures of a sodium styrene sulfonate/methacrylic acid copolymer and sodium nitrate can be measured simultaneously with good accuracy.

Example 5

A calibration of a sodium styrene sulfonate/maleic acid copolymer and sodium sulfite was prepared in a sample of boiler water blowdown collected from a 600 psi boiler.

Boiler Water Blowdown

| Total Hardness | 6.1 ppm as $CaCO_3$ |
| Total Iron | 2.4 ppm as Fe |
| pH | 11.0 |
| Sulfite Residual | 17.5 ppm as $SO_3^{2-}$ |

Analytes

```
1     -     sodium styrene sulfonate/maleic acid copolymer
2     -     sodium sulfite
Concentration ranges in the learning set
      -     polymer   0-25 ppm
      -     sulfite   17.5-44.5 ppm (as SO₃²⁻)
Number of samples in the learning set   -   31
Wavelength range                        -   230-288 nm
```

The test set contained 7 samples of the sodium styrene sulfonate/methacrylic acid and sodium sulfite prepared in the boiler water blowdown.

11

## Table 5

| Sample | Polymer | | | Sulfite | | |
|---|---|---|---|---|---|---|
| | Theoretical Conc. ppm | Predicted Conc. ppm | % Error | Actual* Conc. ppm | Predicted Conc. ppm | % Error |
| 1 | 4.50 | 5.03 | 11.8 | 28.00 | 27.39 | 2.18 |
| 2 | 12.00 | 12.20 | 1.7 | 30.00 | 30.57 | 1.90 |
| 3 | 18.00 | 18.37 | 2.1 | 38.00 | 37.89 | 0.29 |
| 4 | 7.00 | 7.01 | 0.1 | 21.50 | 22.11 | 2.84 |
| 5 | 10.00 | 9.81 | 1.9 | 17.50 | 17.65 | 0.86 |
| 6 | 24.00 | 23.88 | 0.5 | 30.50 | 30.39 | 0.36 |
| 7 | 15.00 | 15.08 | 0.5 | 24.50 | 23.95 | 2.24 |

* The actual concentration of sulfite was determined by iodometric titration

This example shows that mixtures of the sodium styrene sulfonate/maleic acid copolyer and sodium sulfite can be measured simultaneously with good accuracy in a sample of boiler water blowdown.

Example 6

A calibration of a sodium sulfonate/methacrylic acid copolymer and a sodium alkylnaphthalenesulphonate polymer was prepared in a synthetic demineralized feed water containing 10 ppm disodium

phosphate (as $PO_4^{3-}$) at pH 10.

| Number of samples in the learning set | 30 |
|---|---|
| Wavelength range | analyte 1 230-288 nm<br>analyte 2 230-317 nm |
| Concentration ranges in the learning set | analyte 1 0-25 ppm active<br>analyte 2 0-7 ppm active |

The test set contained 5 samples of mixtures of a sodium styrene sulfonate/methacrylic acid copolymer and a sodium alkylnaphthalenesulphonate polymer.

Table 6

| Sample | Analyte 1 | | | Analyte 2 | | |
|---|---|---|---|---|---|---|
| | Theoretical Conc. ppm | Predicted Conc. ppm | % Error | Theoretical Conc. ppm | Predicted Conc. ppm | % Error |
| 1 | 1.00 | 1.12 | 12.0 | 6.00 | 6.08 | 1.3 |
| 2 | 4.00 | 3.95 | 1.3 | 0.50 | 0.53 | 6.0 |
| 3 | 9.00 | 8.79 | 2.3 | 3.40 | 3.40 | 0.0 |
| 4 | 14.40 | 14.33 | 0.5 | 1.90 | 1.90 | 0.0 |
| 5 | 21.00 | 21.22 | 1.0 | 2.60 | 2.58 | 0.8 |

This example demonstrates that the concentration of two polymers can be measured simultaneously with good accuracy in a synthetic demineralized feed water.

Example 7

A calibration of sodium sulfonate/methacrylic acid copolymer and a polymer of sodium alkylnaphthalenesulphonate was prepared in zeolite softened water. 10 ppm disodium phosphate was added and the

14

pH was adjusted to 10.0.

Analysis of Zeolite Water

| Total hardness | 7.3 ppm as $CaCO_3$ |
|---|---|
| Copper | 30 ppb as Cu |
| Iron | <50 ppb as Fe |
| Silica | 7.5 ppm as $SiO_2$ |

Analytes

1. Sodium styrene sulfonate/methacrylic acid copolymer.
2. Sodium alkylnaphthalenesulphonate polymer.

| Concentration ranges in the learning set | |
|---|---|
| Analyte 1 | 0-25 ppm active |
| Analyte 2 | 0-7 ppm active |

| Wavelength range | |
|---|---|
| Analyte 1 | 240-298 nm |
| Analyte 2 | 240-327 nm |

The test set contained 5 samples of mixtures of sodium styrene sulfonate/methacrylic acid copolymer and a sodium alkylnaphthalenesulphonate polymer.

Table 7

| Sample | Analyte 1 | | | Analyte 2 | | |
|---|---|---|---|---|---|---|
| | Theoretical Conc. ppm | Predicted Conc. ppm | % Error | Theoretical Conc. ppm | Predicted Conc. ppm | % Error |
| 1 | 1.00 | 1.25 | 25.0 | 6.00 | 5.98 | 0.3 |
| 2 | 4.00 | 3.94 | 1.5 | 0.50 | 0.51 | 2.0 |
| 3 | 9.00 | 8.93 | 0.7 | 3.40 | 3.36 | 1.2 |
| 4 | 14.40 | 14.01 | 2.7 | 1.90 | 1.87 | 1.6 |
| 5 | 21.00 | 20.33 | 3.2 | 2.60 | 2.63 | 1.2 |

This example demonstrates that the concentration of both polymers can be measured simultaneously with good accuracy in zeolite softened feed water.

## Claims

1. A method for directly measuring the concentration of one or more components of water treatment compositions in a steam generating system which comprises directly determining an absorbance or emission spectrum of the system water in the wavelenth range of from 200 to 2500 nm, and applying chemometrics algorithms to the absorbance or emission spectrum to determine the concentration of the water treatment compositions.

2. A method according to Claim 1 wherein the components of the water treatment compositions are selected from the group consisting of oxygen scavengers, oxygen scavenger catalysts, corrosion inhibitors, scale inhibitors, chelants, nitrates, tracers, neutralizing amines, polymers and mixtures thereof.

3. A method according to Claim 1 wherein the steam generating system is selected from the group consisting of boiler systems, desalinization units, evaporators, turbines and cookers.

4. A method according to Claim 2 wherein the oxygen scavenger is selected from the group consisting of sulfite, hydrazine, erythorbic acid, alkyl hydroxylamines, hydroxyalkyl substituted hydroxylamines and precursors to hydroxylamines.

5. A method according to Claim 4 wherein the alkyl hydroxylamine and precursors to hydroxylamine are selected from the group consisting of diethylhydroxyl amine, n-isopropylhydroxylamine, methylethylketoxime, bis(2-hydroxyethyl)-hydroxylamine, bis(2-hydroxypropyl)-hydroxylamine and bis(2-hydroxybutyl)hydroxylamine.

6. A method according to Claim 2 wherein the oxygen scavenger catalyst is selected from the group consisting of copper, hydroquinone, benzoquinone, 1,2-naphthoquinone-4-sulfonicacid, pyrogallol and t-butylcatechol.

7. A method of determining cycles of concentration in a steam generating system containing nitrate in the system water comprising monitoring the concentration of nitrate in the system water in a feed water stream and in a blowdown stream according to Claim 1 and determining a ratio of the concentration of nitrate in the blowdown to the concentration of nitrate in the feedwater wherein said ratio equals the cycles of concentration.

8. A method of determining cycles of concentration in a steam generating system containing a tracer in the system water comprising monitoring the concentration of tracer in the system water in a feed water stream and in a blowdown stream according to Claim 1 and determining a ratio of the concentration of tracer in the blowdown to the concentration of tracer in the feedwater wherein said ratio equals the cycles of concentration.

9. A method of determining polymer dosage requirements in a steam generating system which is treated with said polymers comprising 1) determining the concentration of hardness of the feedwater with a hardness analyzer which generates a voltage analog which is proportional to the degree of hardness, 2) measuring the polymer treatment concentration in accordance with the method of Claim 1 and generating a voltage analog which is proportional to the concentration, 3) outputting the respective voltage analogs to a logic controller which is capable of calculating a ratio of these voltage analogs, and then comparing the ratio to a predetermined standard operating range, and if the ratio is outside the standard operating range, increasing or decreasing the rate of polymer treatment feed until an acceptable ratio is obtained.

10. A method of determining polymer loss in a steam generating system having a feed water stream and a blowdown stream and which is treated with said polymers comprising measuring the concentration of polymer in the blowdown stream in accordance with the method of Claim 1, measuring the concentration of polymer in the feedwater stream in accordance with the method of Claim 1, determining a value for cycles of concentration and multiplying this value by the concentration of polymer in the feedwater to obtain a theoretical polymer concentration in the blowdown, and then subtracting the concentration of polymer in the blowdown from the theoretical concentration of polymer in the blowdown to obtain the polymer loss.

11. A method of determining polymer loss in a steam generating system which is treated with said polymers in combination with an inert tracer in a known proportion of polymer to tracer said system having a blowdown stream comprising measuring the concentration of polymer in the blowdown stream in accordance with the method of Claim 1, measuring the concentration of tracer in the blowdown stream in accordance with the method of Claim 1, multiplying the proportion of polymer to tracer by the tracer concentration to obtain theoretical concentration of polymer, and then subtracting the concentration of polymer in the blowdown from the theoretical concentration of polymer in the blowdown to obtain the polymer loss.

12. A method according to Claim 2 wherein the polymer is a homopolymer or copolymer containing recurring units selected from the group consisting of acrylates, methacrylates, acrylamides, methacrylamides, sulfonated styrenes, lignosulfonates, tannins, alkyl naphthalene sulfonates, and mixtures thereof.

13. A method according to Claim 8 wherein the tracer is selected from the group consisting of bromophenol, rhodamine, stilbene derivatives, 2-naphthalene sulfonic acid, lissamine FF, sulfostyrene derivatives, propene, xanthene, fluoroscein, and mixtures thereof.

14. A method according to Claim 11 wherein the tracer is selected from the group consisting of bromophenol, rhodamine, stilbene derivatives, 2-naphthalene sulfonic acid, lissamine FF, sulfostyrene derivatives, propene, xanthene, fluoroscein, and mixtures thereof.

# FIG. I

PHOTODIODE
ARRAY

SPECTRUM

DISPLAY

MICROPROCESSOR

FIBER
OPTIC
CABLE

HOLOGRAPHIC
GRATING

FLOW CELL

FLASH
LAMP

ON-LINE
SAMPLE

EP 0 614 085 A1

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | EP-A-0 469 773 (NALCO CHEMICAL) 5 February 1992 PAGES 4-5,13 | 1-3 | G01N33/18 G01N21/31 |
| A | EP-A-0 320 086 (DUBOIS CHEMICALS) 14 June 1989 * page 2 - page 4 * | 1-3 | |
| A | US-A-5 171 450 (HOOTS) 15 December 1992 * column 8 - column 10 * | 1-3,9 | |
| A | US-A-5 006 311 (HOOTS) 9 April 1991 * column 5 - column 7 * | 1,8 | |
| A | EP-A-0 344 694 (HERCULES) 6 December 1989 * page 1 - page 2 * | 1 | |
| A | EP-A-0 522 988 (GIE ANJOU RECHERCHE) 13 January 1993 * page 3 - page 4 * | 1 | |
| A | EP-A-0 418 799 (KURASHIKI BOSEKI) 27 March 1991 * page 3 - page 4 * | 1 | TECHNICAL FIELDS SEARCHED (Int.Cl.5) G01N |
| X,P | EP-A-0 559 305 (GRACE & CO.) 8 September 1993 WHOLE DOCUMENT | 1-10 | |

The present search report has been drawn up for all claims

| Place of search | Date of completion of the search | Examiner |
|---|---|---|
| THE HAGUE | 15 June 1994 | Boehm, C |

EPO FORM 1503 03.82 (P04C01)